# EUROPEAN PATENT SPECIFICATION

(11) **EP 3 728 648 B1**
(45) Date of publication and mention of the grant of the patent: **27.05.2026**
(21) Application number: 18836464.0
(22) Date of filing: 18.12.2018
(51) Int. Cl.: C12N 1/205, A23C 9/123

(54) **FERMENTED DAIRY COMPOSITIONS AND METHODS OF PREPARING THE SAME**
GEGORENE MILCHZUSAMMENSETZUNGEN UND VERFAHREN ZUR HERSTELLUNG DAVON
COMPOSITIONS LAITIÈRES FERMENTÉES ET PROCÉDÉS DE PRÉPARATION DE CELLES-CI

(30) Priority: 19.12.2017 US 201762607681 P
(43) Date of publication of application: 28.10.2020
(73) Proprietor: Compagnie Gervais Danone, 75009 Paris (FR)
(72) Inventor: CAPRONNIER, Sandrine, 91300 Massy (FR); GARAULT, Peggy, 91310 Montlhery (FR); MARCHAL, Laurent, 91360 Villemoisson-sur-Orge (FR); LARRERE, Fanny, 77123 Noisy-sur-École (FR)
(74) Representative: Gevers & Orès
(86) International application number: PCT/EP2018/085521
(87) International publication number: WO 2019/121698

(56) References cited:
- EP-A1- 0 148 299
- EP-A1- 2 119 365
- WO-A1-01/45722
- WO-A1-2004/039964
- WO-A1-2010/058294
- US-A- 4 734 361
- US-A- 5 416 020

## Description

### Field of the Invention

The present invention relates to compositions comprising *L. acidophilus,* and methods of preparing fermented dairy compositions comprising *L. acidophilus* and low levels of D-lactate.

### Technical Background

L-lactate and D-lactate are optical isomers of 2-hydroxypropanoic acid. The L-lactate form is a product of pyruvic acid metabolism that is usually metabolized to pyruvate in the liver and kidneys. D-lactate is a byproduct of bacterial metabolism that is not metabolized by humans. Elevated levels of d-lactate have been associated with metabolic acidosis, a rare condition in humans, but more common in ruminants.

*L. acidophilus* is a gram positive species of bacteria that is a common constituent of the human microbiota. Strains of the species are amongst the most commonly used "probiotics" with a well-established history of safe use. Nonetheless, *L. acidophilus* is also a D-lactate producing species and bacterial causes have been associated with extremely rare instances of d-lactic acidosis. Although commercially used strains of *L. acidophilus* are generally recognized as safe, in certain cases it may be useful to have the possibility to use L-lactate producing strains *(see e.g.,* World Health Organization Codex Alimentarius International Food Standard 72-1981) for example in infant formula.

*L. acidophilus* CNCM I-2273 is briefly mentioned in patent application WO2001045722. The application further discloses fermented milk preparation comprising strains sensitive to the action of mutanolysin, however, the strain is not identified as being susceptible to said enzyme. The strain was further disclosed in patent application WO 2010/058294 where it was stated that "I-2273 strain is a strain which has a blood-cholesterol-lowering activity *in vitro,* but which cannot be readily used for obtaining fermented milk products since it grows very poorly on milk." Thus, although the strain is known in the art, a person of skill in the art would not use it in the preparation of fermented milk products.

EP0148299 A1 and US4734361 A disclose the production of fermented milk product.

### Summary of Various Embodiments

Dairy compositions, inoculums and methods comprising *L. acidophilus* are disclosed. In particular, the inventors unexpectedly found that *L. acidophilus* CNCM I-2273, when used for the preparation of fermented milk compositions, provides a composition having surprisingly low levels of D-lactate.

In a first aspect, the present invention provides a fermented dairy composition comprising L. acidophilus CNCM 1-2273, S. thermophilus and up to 0.02% w/w D-lactate and having a pH equal to or lower than 5.

In a second aspect, the present invention provides a process for the preparation of a fermented dairy composition comprising fermenting a mixture comprising a dairy composition and *L. acidophilus* CNCM I-2273 and *S. thermophilus* to obtain a fermented dairy composition.

### Detailed Description of Various Embodiments

As used herein, the term "ppm" shall be taken to mean "parts per million" One gram in 1 liter is 1000 ppm and one thousandth of a gram (0.001g) in 1 liter is one ppm.

As used herein, the term "x% (w/w)" "x% w/w" is equivalent to "x g per 100 g". Thus, 200 ppm is equivalent to 0.02% w/w. Unless indicated otherwise, all % value shall be taken to indicate x% w/w.

As used herein, the terms "dairy composition", "milk-based composition" or "dairy product" shall be taken to mean a product or composition comprising, comprising essentially of or consisting of milk or milk components and optionally further ingredients.

As used herein, the term "fermented dairy" shall be taken to mean a product or composition that is the product of the acidifying fermentation of a milk-based composition by a starter culture of fermenting microorganisms, in particular bacteria, preferably lactic acid bacteria. As used herein, the term "fermented milk" shall be taken to mean a product or composition derived from milk by the acidifying action of at least one lactic acid bacterium. Accordingly, as used herein, a fermented dairy composition or product can thus be a fermented milk, such as a yoghurt (e.g., a set, stirred or drink yogurt), or a fresh cheese such as a white cheese or a *"petit-Suisse".* It can be also be a strained fermented milk such as a strained yoghurt (e.g., a concentrated or Greek-style yoghurt).

The term "yogurt" or "yoghurt" as used herein shall be taken to mean fermented milk obtained by the acidifying lactic fermentation of specific thermophilic lactic acid bacteria such as *Lactobacillus delbrueckii* subsp. *bulgaricus* and *Streptococcus thermophilus* (also referred to as *Streptococcus salivarius subsp. thermophilus*), which must be viable in the finished product at a minimum CFU. In certain countries, regulations allow the addition of further lactic acid bacteria to yoghurt such as but not limited to strains of *Bifidobacterium* and/or *Lactobacillus acidophilus* and /or *Lactobacillus casei.* These additional lactic acid bacteria strains are intended to impart various properties to the finished product, such as that of providing organoleptic qualities, favoring equilibrium of intestinal flora or modulating the immune system.

As used herein, the term "strained fermented dairy composition" shall be taken to mean a fermented dairy composition which has been subjected to a post-fermentation acid whey separation process.

As used herein, the term "spoonable" shall be taken to mean a solid or semi-solid that may be consumed by means of a spoon or other utensil.

As used herein, the term "fermentation" shall be taken to mean the metabolism of a substance by microorganisms, e.g. bacteria, yeasts, or other microorganisms.

As used herein, the term "cfu" or "CFU" shall be taken to be an abbreviation of the term "colony forming unit".

As used herein, the term "CNCM I-" followed by a 4 digit number shall be taken to refer to a strain deposited at the Collection Nationale de Cultures de Microorganismes (CNCM) 25 rue du Docteur Roux, Paris, France under the Budapest Treaty with an accession number corresponding to said 4 digit number, e.g. CNCM I-2273. CNCM I-2273 has been deposited at the Collection Nationale de Cultures de Microorganismes (CNCM) (Institut Pasteur, 25 Rue du Docteur Roux, Paris, France) under the Budapest Treaty on Aug. 3, 1999. The deposit was made in accordance with the Budapest Treaty on the International Recognition of the Deposit of Microorganisms for the Purposes of Patent Procedure, as provided therein the applicant requests that a sample of the deposited micro-organisms only be made available to an independent expert, until the date on which the patent may be granted.

As used herein, reference to a bacterial strain or species shall be taken to include functionally equivalent bacteria derived therefrom such as but not limited to mutants, variants or genetically transformed bacteria. These mutants or genetically transformed strains can be strains wherein one or more endogenous gene(s) of the parent strain has (have) been mutated, for instance to modify some of their metabolic properties (e.g., their ability to ferment sugars, their resistance to acidity, their survival to transport in the gastrointestinal tract, their post-acidification properties or their metabolite production). They can also be strains resulting from the genetic transformation of the parent strain to add one or more gene(s) of interest, for instance in order to give to said genetically transformed strains additional physiological features, or to allow them to express proteins of therapeutic or prophylactic interest that one wishes to administer through said strains. These mutants or genetically transformed strains can be obtained from the parent strain by means of conventional techniques for random or site-directed mutagenesis and genetic transformation of bacteria, or by means of the technique known as "genome shuffling". In the present text, strains, mutants and variants derived from a parent species or strain will be considered as being encompassed by reference to said parent species or strain, e.g. the phrases *"Streptococcus thermophilus" and* "CNCM I-2273" shall be taken to include strains, mutants and variants derived therefrom. Accordingly, as used herein reference to a bacterial strain specified by an accession or deposit number shall be taken to encompass variants thereof having at least 95 % identity (see: Stackebrandt & Goebel, 1994, Int. J. Syst. Bacteriol. 44:846-849). In a particularly preferred embodiment, said variant has at least 97 % identity with the 16S rRNA sequence of said specified strain, more preferably at least 98 % identity, more preferably at least 99 % or more identity.

As used herein, the term "substantially pure" when used in reference to a bacterial strain refers to the percent of said bacterial strain relative to the total micro-organism content. Substantially pure can be at least about 99.99%, at least about 99.90%, at least about 99.50%, at least about 99.00%, at least about 95.00%, at least about 90.00%, at least about 85.00%, or at least about 75.00%.

As used herein, a "lactic acid bacterium" is a Gram-positive, acid-tolerant, generally non-sporulating and non-respiring, either rod- or cocci-shaped bacterium that is able to ferment sugars into lactic acid.

The present invention relates to dairy compositions, inoculums and methods comprising *L. acidophilus.*

### Dairy Compositions.

In a first aspect, the present invention provides fermented dairy compositions comprising L. acidophilus CNCM 1-2273, S. thermophilus and up to 0.02% w/w D-lactate and having a pH equal to or lower than 5.

It is preferred that said D-lactate is present in quantities of at least 1 ppm (parts per million), at least 10 ppm, at least 20 ppm or at least 50 ppm. Accordingly in preferred embodiments the amount of D-lactate is 1 ppm - 200 ppm, such as 10 ppm - 200 ppm, 20 ppm - 200 ppm or 50 ppm - 200 ppm. The fermented dairy composition according to the various embodiments of the invention are suitable to be administered to infants or young children, preferably starting from the age of 4 months.
The fermented dairy compositions according to embodiments of the invention preferably comprise at least 10⁶,10⁷ , 10⁸ or 10⁹ CFU/g *L. acidophilus,* preferably at least 10⁶,10⁷ , 10⁸ or 10⁹ CFU/g *L. acidophilus,* preferably strain CNCM 1-2273.

In a preferred embodiment fermented dairy compositions of the invention comprise less than or egual to 1x 10⁸ CFU/g, more preferably 2.5, 5 or 7.5 x 10⁷ CFU/g *L. acidophilus,* preferably strain CNCM I-2273.

In a preferred embodiment fermented dairy compositions of the invention comprise more than or egual to 8 x 10⁶ CFU/g, more preferably 8.1, 8.3, 8.5 or 8.7 x 10⁶ 1 x 10⁷ CFU/g L. *acidophilus,* preferably strain CNCM I-2273.

In embodiments fermented dairy compositions of the invention comprise between 8x10⁶ and 1x 10⁸ cfu/g or 8.7 x10⁶and 2.5 x 10⁸ cfu/g *L. acidophilus,* preferably strain CNCM I-2273.

The fermented dairy compositions according to embodiments of the invention preferably further comprises at least one strain of *L. bulgaricus.*

The dairy composition of the invention comprises milk, preferably fermented milk. Preferably, the composition comprises at least about 30 % (w/w) milk, more preferably at least about 50% (w/w) milk and even more preferably at least about 70% (w/w) milk. In embodiments, the composition comprises at 30 % to 100% (w/w) milk. In other embodiments, the composition comprises 50% to 100% (w/w) milk. In other embodiments, the composition comprises 70% to 100% (w/w) milk. Preferably, the milk is vegetal and/or animal milk, more preferably soya, almond, oat, hemp, spelt, coconut, rice, goat, ewe, camel, mare or cow milk, and most preferably to cow milk. Preferably, the milk(s) are heat-treated, typically pasteurized, to ensure sterility. Preferably, the heat treatment is carried out prior to the preparation of the fermented dairy composition.

Preferably, the milk comprises one or more of skimmed, partially-skimmed or non-skimmed milk. Preferably, the milk or milks may be in liquid, powdered and/or concentrated form. In one embodiment, the milk further comprises milk components preferably selected from the group consisting of cream, casein, caseinate (for example calcium or sodium caseinate), whey proteins notably in the form of a concentrate (WPC), milk proteins in the form of a concentrate (MPC), milk protein hydrolysates, and mixtures thereof. In one embodiment, the mixture further comprises plant and/or fruit juices. In one embodiment, the milk or milks may be enriched or fortified with further milk components or other nutrients such as but not limited to vitamins, minerals, trace elements or other micronutrients.

Preferably, the dairy composition comprises above about 0.3 g per 100 g by weight free lactic acid, more preferably above about 0.7 g or 0.6 g per 100 g by weight free lactic acid. In embodiments, the composition comprises 0.3 g to 0.7 grams per 100 g by weight free lactic acid.

Preferably, the dairy composition comprises a protein content at least equivalent to that of the milk or milks from which it is derived, preferably at least about 2.5%, more preferably at least about 3% or 3.5% (w/w). The composition has a pH equal to or lower than 5, preferably between about 3 and about 4.5, and more preferably between about 3.5 and about 4.5, alternatively between about 3 and about 4.7.

Preferably, the the dairy composition has a viscosity lower than 200 mPa.s, more preferably lower than 100 mPa.s and most preferably lower that 60 mPa.s, at 10°C, at a shear rate of 64 s⁻¹. In other embodiments, the composition has a viscosity range of 1 to 200 mPa.s, 1 to 100 mPa.s, or 1 to 60 mPa.s, at 10°C, at a shear rate of 64 s⁻¹. In other embodiments, the composition has a viscosity range of 10 to 200 mPa.s, 10 to 100 mPa.s, or 10 to 60 mPa.s, at 10°C, at a shear rate of 64 s⁻¹. In other embodiments, the composition has a viscosity range of 30 to 200 mPa.s, 30 to 100 mPa.s, or 30 to 60 mPa.s, at 10°C, at a shear rate of 64 s⁻¹.

The fermented dairy composition according to embodiments of the invention is preferably a product selected from the group comprising yogurt, set yogurt, stirred yogurt, pourable yogurt, yogurt drink, frozen yogurt, kefir, buttermilk, quark, sour cream, fresh cheese and cheese. In one embodiment, the composition is a drinkable composition, more preferably a fermented milk drink such as but not limited to a yogurt drink, kefir etc. In an alternative embodiment, the composition is a composition that is spoonable, such as a set or stirred yogurt or equivalent thereof.

In one embodiment, the fermented dairy composition is a strained fermented dairy composition. The strained fermented dairy composition preferably has the following contents (% by weight):
- from 8.5% to 11.0% of milk protein
- from 0.0% to 8.0% of fat, for example from 0.0% to 3.5% or from 3.5% to 8.0%
- from 0.00% to 4.20% of lactose, for example from 2.80% to 4.20%

The pH of the strained fermented dairy composition can for example be of from 3.80 to 4.65.

Preferably, the composition, according to embodiments of the invention, may be stored, transported and/or distributed at a temperature of from 1°C to 10°C for at least about 30 days, at least about 60 days or at least about 90 days from packaging and remain suitable for consumption.

In other embodiments, the dairy compositions comprise further lactic acid bacteria. Examples of lactic acid bacteria that can be used include but are not limited to *Lactobacilli* (for example *Lactobacillus acidophilus, Lactobacillus buchneri, Lactobacillus delbruckeii,* in particular *L. delbrueckii subsp. bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus*); *Lactococci* (for example *Lactococcus lactis,* typically *Lactococcus lactis subsp. lactis* or *Lactococcus lactis* subsp. *cremoris*). Typically, a mixture or association of a plurality of species of lactic acid bacteria may be used, such as a mixture or association of *Lactobacillus* and *Streptococcus.* For the preparation of yogurt, this typically includes *Lactobacillus bulgaricus* (also referred to as *Lactobacillus delbrueckii* subsp. *bulgaricus*) and *Streptococcus thermophilus,* optionally with additional microorganisms such as but not limited to probiotic species or other species that may provide desirable organoleptic or other qualities to the composition, e.g. *Lactococcus lactis.*

Accordingly, in one embodiment, the composition further comprises at least one strain of *Lactobacillus* bulgaricus. In other embodiments, the dairy compositions of the invention comprise at least 10⁵ cfu/g,more preferably at least 10⁶ cfu/g, such as at least 10⁷ cfu/g, e.g. at least 10⁸ cfu/g, such as at least 10⁹ cfu/g, e.g. at least 10¹⁰ cfu/g, such as at least 10¹¹ cfu/g of each bacterial strain per gram of dairy composition. In other embodiments, the dairy compositions of the invention comprise 10⁵ to 10¹² or 10⁶ to 10¹⁰ colony forming unit (CFU) *L. acidophilus CNCM 1-2273* per gram of composition. In a most preferred embodiment the dairy compositions comprise between 1x10⁶ and 2x 10⁷ cfu/g *L. acidophilus,* preferably strain CNCM I-2273.

Preferably, the composition is a packaged product that comprises at least 10⁶, more preferably at least 10⁷ and most preferably at least 10⁸ colony forming unit (CFU) *L. acidophilus,* preferably strain *CNCM I-2273* per gram (g) of composition subsequent to storage, transport and/or distribution at a temperature of from 1°C to 10°C for at least about 30 days, at least about 60 days or at least about 90 days from packaging.

In other embodiments, the composition is a packaged product that comprises 10⁵ to 10¹² or 10⁶ to 10¹⁰ colony forming unit (CFU) *L. acidophilus CNCM 1-2273* per gram (g) of composition subsequent to storage, transport and/or distribution at a temperature of from 1°C to 10°C for at least about 30 days, at least about 60 days or at least about 90 days from packaging.

In other embodiments, the dairy composition further comprises an intermediate preparation. They are typically used to modify the taste, mouthfeel and/or texture of a dairy composition, for example of a fermented dairy composition. They can used also to introduce some additives such as nutrients. They typically comprise sweetening agents, flavors, color modifiers, cereals and/or fruit. Intermediate fruit preparations are for example slurries or fruit preparations. Flavors include for example fruit flavors, vanilla flavors, caramel flavors, coffee flavors, chocolate flavors.

Fruit preparations typically comprise fruits, as used herein the term "fruit" refers to any fruit form, including for example full fruits, pieces, purees, concentrates, juices etc.

The intermediate preparation or slurry typically comprises a stabilizing agent, having at least one stabilizer. The stabilizing agent can comprise at least two stabilizers. Such stabilizers are known to the one skilled in the art. They typically help in avoiding phase separation of solids, for examples of fruits or fruits extracts and/or in avoiding syneresis. They typically provide some viscosity to the composition, for example a viscosity (Bostwick viscosity at 20°C) of from 1 to 20 cm/min, preferably of from 4 to 12 cm/min.

The stabilizing system or the stabilizer can for example be a starch, a pectin, a guar, a xanthan, a carrageenan, a locust bean gum, or a mixture thereof. The amount of stabilizing system is typically of from 0.5 to 5% by weight.

The intermediate preparation can typically comprise organoleptic modifiers. Such ingredients are known by the one skilled in the art.

The organoleptic modifiers can be for example sweetening agents different from sugar, coloring agents, cereals and/or cereal extracts.

Examples of sweetening agents are ingredients referred to as High Intensity Sweeteners, such as sucralose, acesulfamK, aspartam, saccharine.

Examples of fruits include for example strawberry, peach, apricot, mango, apple, pear, raspberry, blueberry, blackberry, passion, cherry, and mixtures or associations thereof, such as peach-passion.

The fruits can be for example provided as:
- frozen fruit cubes, for example 10 mm fruit cubes, for example Individual Quick Frozen fruit cubes, for example strawberry, peach, apricot, mango, apple, pear fruit cubes or mixtures thereof,
- Aseptic fruit cubes, for example 10 mm fruit cubes, for example strawberry, peach, apricot, mango, apple or pear fruit cubes or mixtures thereof,
- fruit purees, for example fruit purees concentrated from 2 to 5 times, preferably 3 times, for example aseptic fruit purees, for example strawberry, peach, apricot, mango, raspberry, blueberry or apple fruit purees or mixtures thereof,
- single aseptic fruit purees, for example strawberry, raspberry, peach, apricot, blueberry or apple single aseptic fruit purees or mixture thereof,
- frozen whole fruits, for example Individual Quick Frozen whole fruits, for example blueberry, raspberry or blackberry frozen whole fruits, or mixtures thereof,
- mixtures thereof.

The ingredients and/or components of the intermediate preparation and the amounts thereof can be typically such that the composition has a brix degree of from 1 to 65 brix, for example from 1 to 10 brix, or from 10 to 15 brix, or from 15 to 20 brix, or from 20 to 25 brix, or from 25 to 30 brix, or from 30 to 35 brix, or from 35 to 40 brix, or from 40 to 45 brix, or from 45 to 50 brix, or from 50 to 55 brix, or from 55 to 60 brix, or from 55 to 60 brix, or from 60 to 65 brix.

A fruit preparation can for example comprise fruit in an amount of from 30% to 80% by weight, for example from 50 to 70% by weight.

The intermediate preparation can comprise water. It is mentioned that a part of the water can come from ingredients used to prepare the fruit preparation, for example from fruits or fruit extracts or from a phosphoric acid solution.

The fruit preparation can comprise pH modification agents such as citric acid. The fruit preparation can have a pH of from 2.5 to 5, preferably of from 2.8 to 4.2.

Typically a fruit preparation can be added in an amount of 5-35% by weight with reference to the total amount of composition. In embodiments the composition of the invention comprises up to about 30% (w/w) of said intermediate preparation, e.g., up to about 10%, 15%, 20%, 25% (w/w). In one embodiment, the composition according to embodiments of the invention comprise 1% to 30% (w/w) of said intermediate preparation. In alternative embodiments, the composition comprises 1% to 25% (w/w) of said intermediate preparation. In further alternative embodiments, the composition comprises 1% to 20% (w/w) of said intermediate preparation. In additional embodiments, the composition comprises 1% to 15% (w/w) of said intermediate preparation. In further additional embodiments, the composition comprises 1% to 10% (w/w) of said intermediate preparation.

Preferably, the composition, according to embodiments of the invention is provided in a sealed or sealable container containing about 50 g, 60 g, 70 g, 75 g, 80 g, 85 g, 90 g, 95 g, 100 g, 105 g, 110 g, 115 g, 120 g, 125 g, 130 g, 135 g, 140 g, 145 g, 150 g, 200 g, 300 g, 320 g or 500 g or about 1 oz, 2 oz, 3 oz, 4 oz, 5 oz, 6 oz or 12 oz product by weight.

In other embodiments, the composition is provided in a sealed or sealable container containing about 50 g to 500 g, 60 g to 500 g, 70 g to 500 g, 75 g to 500 g, 80 g to 500 g , 85 g to 500 g, 90 g to 500 g, 95 g to 500 g, 100 g to 500 g, 105 g to 500 g, 110 g to 500 g, 115 g to 500 g, 120 g to 500 g, 125 g to 500 g, 130 g to 500 g, 135 g to 500 g, 140 g to 500 g, 145 g to 500 g, 150 g to 500 g, 200 g to 500 g, 300 g to 500 g, 320 g to 500 g or 500 g product by weight. In other embodiments, the composition is provided in a sealed or sealable container containing about 1 oz to 12 oz, 2 oz to 12 oz, 3 oz to 12 oz, 4 oz to 12 oz, 5 oz to 12 oz, 6 oz to 12 oz or 12 oz product by weight.

### Inoculum Compositions

The bacterium as described herein is useful as starter culture in the preparation of food compositions, such as fermented dairy products. Accordingly, in an aspect the present disclosure provides an inoculum comprising *L. acidophilus* CNCM I-2273 that is suitable for the preparation of fermented dairy compositions. The inoculum is suitable for the direct inoculation of *L. acidophilus* CNCM I-2273 into a composition comprising milk to provide fermented dairy compositions of the invention, typically without the need for a culture step prior to the said direct inoculation.

Typically, the inoculum further comprises excipient or carriers, the selection of which is within the scope of the skilled person but may include buffers or culture media. The inoculum may optionally comprise further components such as cryoprotectants, preservatives and/or additives including nutrients such as yeast extracts, cysteine, sugars and vitamins.

Typically, the inoculum is for use in the preparation of fermented dairy products, according in one embodiment the inoculum of the invention may be provided to the dairy composition in quantities of up to about 500 mg / l.

Typically the inoculum is fresh, frozen, dried or lyophilized. The inoculum may be in liquid, dry, spray-dried or solid form. It is particularly preferred that the inoculum is in liquid form. The inoculum may be defrosted and/or dispersed in liquid (e.g. water) prior to inoculation into a composition comprising milk.

In other embodiments, the inoculum comprises at least 10⁹ cfu, e.g. at least 10¹⁰ cfu, such as at least 10¹¹ cfu *L. acidophilus CNCM 1-2273* per gram of inoculum composition. In other embodiments, the inoculum comprises 10⁹ to 10¹² colony forming unit (CFU) , or more preferably 10¹⁰ to 10¹² colony forming unit (CFU) *L. acidophilus CNCM 1-2273* per gram of inoculum.

Preferably, the inoculum comprising *L. acidophilus CNCM I-2273* is substantially pure.

In a further aspect the present disclosure provides a mixture or kit of parts of the inoculum preferably together with inoculum of further lactic acid bacteria.

Examples of lactic acid bacteria that can be used include but are not limited to *Streptococcus thermophilus, Lactobacilli* (for example *Lactobacillus acidophilus, Lactobacillus buchneri, Lactobacillus delbrueckii,* in particular *L. delbrueckii subsp. bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus*); *Lactococci* (for example *Lactococcus lactis,* typically *Lactococcus lactis subsp. lactis* or *Lactococcus lactis subsp. cremoris*). Preferably, the inoculum mixture further comprises *Lactobacillus* and/or *Streptococcus.* For the preparation of yogurt, the inoculum mixture typically comprises *Lactobacillus bulgaricus* (also referred to as *Lactobacillus delbruckeii subsp. bulgaricus*) and *Streptococcus thermophilus,* optionally with additional microorganisms such as but not limited to probiotic species or other species that may provide desirable organoleptic or other qualities to the composition, e.g. *Lactococcus lactis* or *Lactobacillus acidophilus.* In other embodiments the inoculum of lactic acid bacteria comprises at least 10⁹ cfu, e.g., at least 10¹⁰ cfu, such as at least 10¹¹ cfu of said further lactic acid bacteria per gram of inoculum composition. In embodiments, the inoculum comprises 10⁹ to 10¹² colony forming unit (CFU) , or more preferably 10¹⁰ to 10¹² colony forming unit (CFU) *L. acidophilus CNCM 1-2273* per gram of inoculum.

Accordingly, in one aspect the present disclosure provides an inoculum mixture comprising a L. acidophilus CNCM I-2273 inoculum of the invention and further comprising at least one inoculum of *Streptococcus thermophilus.*

In a further aspect the present disclosure provides an inoculum mixture comprising a *L. acidophilus* CNCM I-2273 inoculum and further comprising at least one inoculum of *Streptococcus thermophilus,* one inoculum of *Lactobacillus bulgaricus* and optionally one or more additional inoculum of *Lactococcus lactis* or *Lactobacillus acidophilus.*

In a further aspect the present disclosure provides an inoculum mixture comprising a *L. acidophilus* CNCM I-2273 inoculum and further comprising at least one inoculum of *Streptococcus thermophilus,* one inoculum of *Lactobacillus bulgaricus* and at least one or more additional inoculum of a *Lactobacillus acidophilus* strain.

### Methods for the Preparation of Fermented Dairy Compositions

The bacteria as provided herein are suitable for use in the preparation of fermented dairy compositions. Accordingly, an aspect, the present disclosure also relates to the intended use of *L. acidophilus,* such as strain CNCM I-2273 for the preparation of a food composition. Thus, in some embodiments, the present disclosure provides processes for the preparation of a fermented dairy composition comprising inoculating a milk-based composition with *L. acidophilus* and fermenting.

In a first aspect the present disclosure provides a process for the preparation of a fermented dairy composition comprising fermenting a mixture comprising a dairy composition and *L. acidophilus* to obtain a fermented dairy composition comprising D-lactate in quantities of up to 200 ppm d-lactate. In an alternative aspect the present disclosure provides the use of *L. acidophilus* in the preparation of a fermented dairy composition comprising D-lactate in quantities of up to 200 ppm D-lactate. It is preferred that said D-lactate is present in quantities of at least 1 ppm (parts per million), at least 10 ppm, at least 20 ppm or at least 50 ppm. It is particularly preferred that said *L. acidophilus* is *L. acidophilus* CNCM I-2273.

It is preferred that said process comprises fermenting a mixture comprising a dairy composition and *L. acidophilus* to obtain a fermented dairy product comprising less than or egual to 1x 10⁸ CFU/g, more preferably 2.5, 5 or 7.5 x 10⁷ CFU/g *L. acidophilus,* preferably strain CNCM I-2273.

It is preferred that said process comprises fermenting a mixture comprising a dairy composition and *L. acidophilus* to obtain a fermented dairy product comprising more than or egual to 8 x 10⁶ CFU/g, more preferably 8.1, 8.3, 8.5 or 8.7 x 10⁶ or 1 x 10⁷ CFU/g *L. acidophilus,* preferably strain CNCM I-2273.

It is preferred that said process comprises fermenting a mixture comprising a dairy composition and *L. acidophilus* to obtain a fermented dairy product comprising between 8x10⁶ and 1x 10⁸ cfu/g; 8.7x10⁶ and 2.5 x 10⁸ cfu/g *L. acidophilus,* preferably strain CNCM I-2273.

The resent invention also provides a process for the preparation of a fermented dairy composition comprising fermenting a mixture comprising a dairy composition and *L. acidophilus* CNCM I-2273 and *S. thermophilus* to obtain a fermented dairy composition. In an alternative embodiment, the present invention provides the use of *L. acidophilus* CNCM I-2273 and *S. thermophilus* in the preparation of a fermented dairy composition. It is particularly preferred that in embodiments of methods or uses of the invention said bacterial strains are in the form of an inoculum or mixture thereof as described according to the present invention.

It is preferred that in embodiments of methods or uses of the invention said fermented dairy composition comprises at least 10⁶,10⁷ , 10⁸ or 10⁹ CFU/g *L. acidophilus.* It is preferred that in embodiments of methods or uses of the invention said mixture comprises at least 10⁷ CFU/g *L. acidophilus.*

It is preferred that in embodiments of methods or uses of the invention said fermented dairy composition comprises less than or egual to 1x 10⁸ CFU/g, more preferably 2.5, 5 or 7.5 x 10⁷ CFU/g *L. acidophilus,* preferably strain CNCM I-2273.

It is preferred that in embodiments of methods or uses of the invention said fermented dairy composition comprises more than or egual to 8.1, 8.3, 8.5 or 8.7 x 10⁶ or 1 x 10⁷ CFU/g, more preferably 8x 10⁶ CFU/g *L. acidophilus,* preferably strain CNCM I-2273.

It is preferred that in embodiments of methods or uses of the invention said fermented dairy composition comprises between 8x10⁶ and 1x 10⁸ cfu/g; 8.7x10⁶ and 2.5 x 10⁸ cfu/g *L. acidophilus,* preferably strain CNCM I-2273.

It is preferred that in embodiments of methods or uses of the invention said mixture further comprises at least one strain of *L. bulgaricus.*

It is preferred that in embodiments of methods or uses of the invention said fermentation is carried out at a temperature of less than about 45°C or 42°C, particularly preferred is a temperature of 35°C-41°C, more preferably 39°C-41°C.

The embodiments of methods or uses may be carried out as a process comprising the following steps:
i) providing a mixture comprising:
   a) milk
   b) *L. acidophilus*
ii) fermentation of said mixture to provide a fermented dairy composition.

Preferably, fermented dairy compositions are prepared using milk that has been subjected to heat treatment at least equivalent to pasteurization. Preferably, the heat treatment is carried out prior to the preparation of the composition.

Typically, milk is pasteurized by means of the following successive steps:
1) standardization of fatty substances of the raw material so as to obtain a standardized substance,
2) enrichment with dried matter of the standardized substance obtained in the preceding stage, so as to obtain an enriched substance,
3) preheating of the enriched substance obtained in the preceding stage, so as to obtain a starting substance,
4) pasteurization and holding of the starting substance obtained in the preceding stage, so as to obtain a pasteurized and held substance,
5) an optional stage of homogenization of the pasteurized and held substance obtained in the preceding stage, so as to obtain a pasteurized, held and optionally homogenized substance,
6) initial cooling of the pasteurized, held and optionally homogenized substance obtained in the preceding stage, so as to obtain a pasteurized starting substance that has been held, optionally homogenized, and cooled down.

As used herein, "standardization of fatty substances" is taken to mean a stage of bringing the quantity of fats present in the starting substance to a pre-determined level. Enrichment with dried matter involves the addition of proteins and fatty substance in order to modify curd firmness.

As used herein, "holding" is taken to mean a rapid heating and maintenance of temperature of the milk and makes it possible to destroy the vegetative microbial flora, including pathogenic forms. Its typical duration is from 4 to 10 minutes, in particular from 5 to 8 minutes, and in particular approximately 6 minutes.

As used herein "homogenization" is taken to mean the dispersion of the fatty substances in the milk-type substance into small fat globules. The homogenization is carried out for example at a pressure of 100 to 280 bars, in particular 100 to 250 bars, in particular 100 to 200 bars, in particular approximately 200 bars. This homogenization stage is purely optional. It is in particular absent from the production process of products with 0% fatty substances.

Typically, a fermented dairy composition is prepared by culture of milks at a suitable temperature with suitable microorganisms to provide a reduction in pH, to a pH equal to or lower than 5, preferably between about 3 and 4.7; more preferably between about 3.5 and about 4.7. The pH can be adjusted by controlling the fermentation by the microorganism and stopping it when appropriate, for example by cooling.

According to a further embodiment of the process for the preparation of a fermented dairy composition as defined above, the mixture comprising milk and *L. acidophilus* (preferably *L. acidophilus* CNCM I-2273) further comprises at least one, two, three or more strains of lactic acid bacteria.

The selection of suitable lactic acid bacteria strains is within the scope of the skilled person and is typically a thermophillic lactic acid bacteria. Examples of lactic acid bacteria that can be used include but are not limited to *Lactobacilli* (for example *Lactobacillus acidophilus, Lactobacillus buchneri, Lactobacillus delbruckeii,* in particular *L. delbrueckii subsp. bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus); Lactococci* (for example *Lactococcus lactis,* typically *Lactococcus lactis subsp. lactis* or *Lactococcus lactis* subsp. *cremoris*). Typically, a mixture or association of a plurality of species of lactic acid bacteria may be used, typically a mixture or association of *Lactobacillus* and *Streptococcus.* For the preparation of yogurt this typically includes *Lactobacillus bulgaricus* (also referred to as *Lactobacillus delbrueckii* subsp. *bulgaricus*) and *Streptococcus thermophilus,* optionally with additional microorganisms such as but not limited to probiotic species or other species that may provide desirable organoleptic or other qualities to the composition, e.g. *Lactococcus lactis.*

Accordingly, in one embodiment, the mixture further comprises at *Lactobacillus bulgaricus,* Streptococcus thermophilus and optionally one or more strains of *Lactococcus lactis.*

Suitable temperatures for milk fermentation are typically about 36°C to about 44°C and the temperature is maintained for an incubation time sufficient to provide the desired reduction in pH. For the preparation of a fermented dairy composition the temperature at the start of fermentation is typically about 36°C to about 43°C, in particular about 37°C to about 40°C, the temperature at the end of fermentation is typically about 37°C to about 44°C, in particular about 38°C to about 41°C. The fermentation time is typically about 6 to about 11 hours.

Subsequent to the fermentation, the fermented milk is cooled. Optionally, a stage of intermediate cooling of the fermented milk may be performed to provide a pre-cooled fermented milk having a temperature of between about 22°C and about 4°C. Typically the intermediate cooling time is about 1 hour to about 4 hours, in particular about 1 hour 30 minutes to about 2 hours. The pre-cooled fermented milk is typically stored for up to 40 hours or less.

Preferably, a stage of final cooling of the fermented milk is performed such that the temperature at the start of the final cooling is less than about 22°C and the temperature at the end of the final cooling is about 4°C to about 10°C. The cooled composition may then be stored, transported and/or distributed at a temperature from about 1°C to about 10°C for at least about 30 days, at least about 60 days or at least about 90 days.

According to a further embodiment, the process for the preparation of a fermented dairy composition as defined above optionally comprises a stage of stirring at a pressure of at least 20 bars, or performing a dynamic smoothing, to obtain a composition having the desired viscosity, typically a viscosity of up to 20 mPa.s. Stirring or dynamic smoothing operations provide some shear to composition that typically allow a viscosity drop. Such operations are known by the one skilled in the art, and can be operated with conventional appropriate equipment. This stage is typically performed at cold temperature, for example at a temperature of form 1 °C to 20°C. Without intending to be bound to any theory, it is believed that applying some shear at cold temperature, typically by stirring at high pressure or by performing a dynamic smoothing, can lead to a fluid gel formation within the composition, that provides improved stability even at a low viscosity of up to 20 mPa.s.

Alternatively, according to a further embodiment, the process for the preparation of a fermented dairy composition as defined above optionally comprises a stage of acid whey removal to provide a "strained fermented dairy composition". In this step an acid whey composition is separated from the curd resulting from the protein coagulation due to acidification during fermentation. Thus one obtains:
- a fermented dairy composition, typically comprising the proteins coagulum, referred to as a strained fermented dairy composition, and
- an acid whey by-product

Such separation steps are known by the one skilled in art, for example in processes of making "greek yogurts". The separation can for example be carried out by reverse osmosis, ultrafiltration, or centrifugal separation. The separation step can be performed for example at a temperature of from 30°C to 45°C.

According to a further embodiment, the process for the preparation of a fermented dairy composition as defined above optionally comprises a stage of addition of an intermediate preparation as described above prior or subsequent to fermentation, said intermediate preparation typically comprising a preparation of fruits and/or cereals and/or additives such as flavorings and/or colourings.

It is preferred that in embodiments of methods or uses of the invention said fermented dairy composition is stored at a temperature of from 1°C to 10°C, preferably under refrigerated conditions for at least 24, 48 or 72 hours after packaging prior to consumption. The product of the invention can typically be used as a food product, more typically as a nutritional or functional food product. Thus, the product of the invention can be typically used by oral administration by a subject. Preferably said subject is a mammal, preferably a human being. In embodiments of the invention said subject is at least 4 months of age young children, preferably starting from the age of 4 months comprising a step to oral administration to the product to a subject. Accordingly in embodiments, the present invention provides the use of a fermented dairy compositions of the inventions as a nutritional compositions by oral administration, preferably to infants or young children, wherein it is particularly preferred that said infants or young children are at least 4 months old.

The invention will be further illustrated by the following non-limiting Figures and Example.

### Description of the Figures

Figure 1 shows milk acidification kinetics of *L. acidophilus* CNCM I-2273 and control strain (strain X) together with *S. thermophilus* in milk. Time (in minutes) is provided on the x-axis, and pH is represented on the y-axis.
Figure 2 shows milk acidification kinetics of *L. acidophilus* strains reference nos. 28, 30, 32, 43 and CNCM I-2273 in milk ("lait"). Time (in minutes) is provided on the x-axis, and pH is represented on the y-axis.
Figure 3 shows milk acidification kinetics of *L. acidophilus* strains reference nos. 28, 30, 32, 43 and CNCM I-2273 in milk ("lait") supplemented with 20g/l glucose. Time (in minutes) is provided on the x-axis, and pH is represented on the y-axis.
Figure 4 shows milk acidification kinetics of *L. acidophilus* strains reference nos. 28, 30, 32, 43 and CNCM I-2273 in milk ("lait") supplemented with 0.5g/l N3 peptide (Vitalarmor 950). Time (in minutes) is provided on the x-axis, and pH is represented on the y-axis.
Figure 5 shows milk acidification kinetics of *L. acidophilus* strains reference nos. 28, 30, 32, 43 and CNCM I-2273 in milk ("lait") supplemented with 2g/l yeast extract (YE). Time (in minutes) is provided on the x-axis, and pH is represented on the y-axis.

### Examples

### Example 1: Screening of Strains

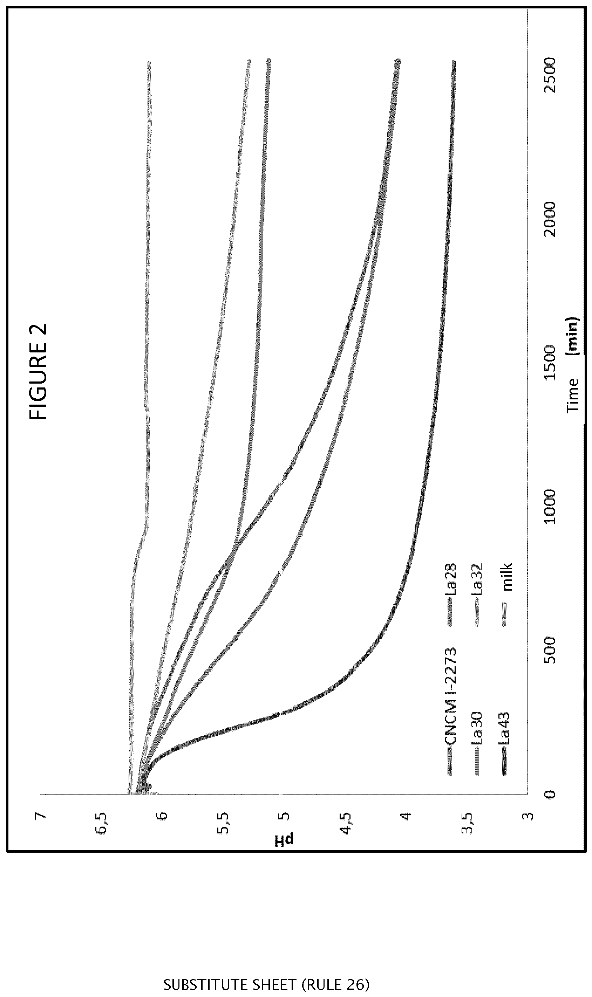

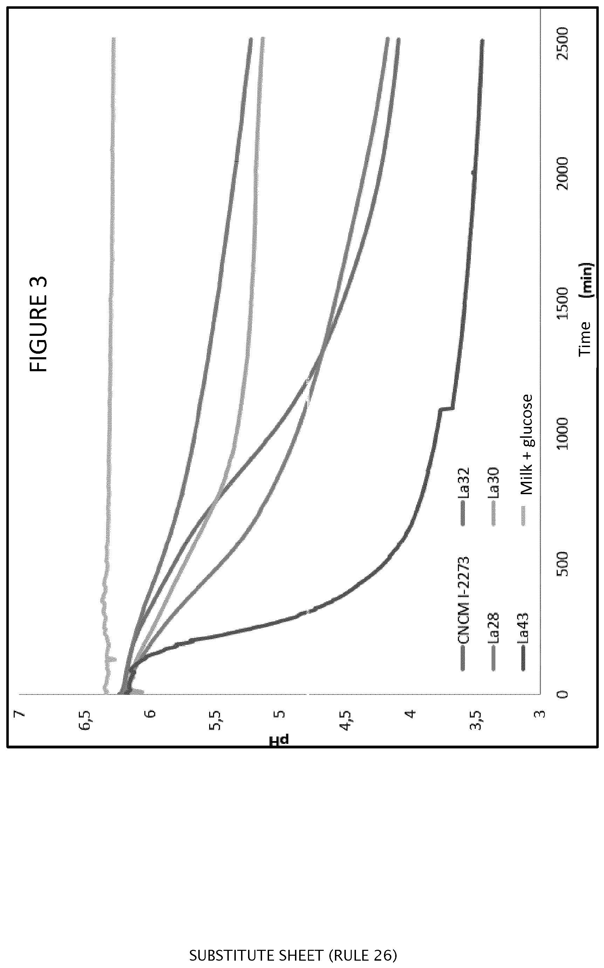

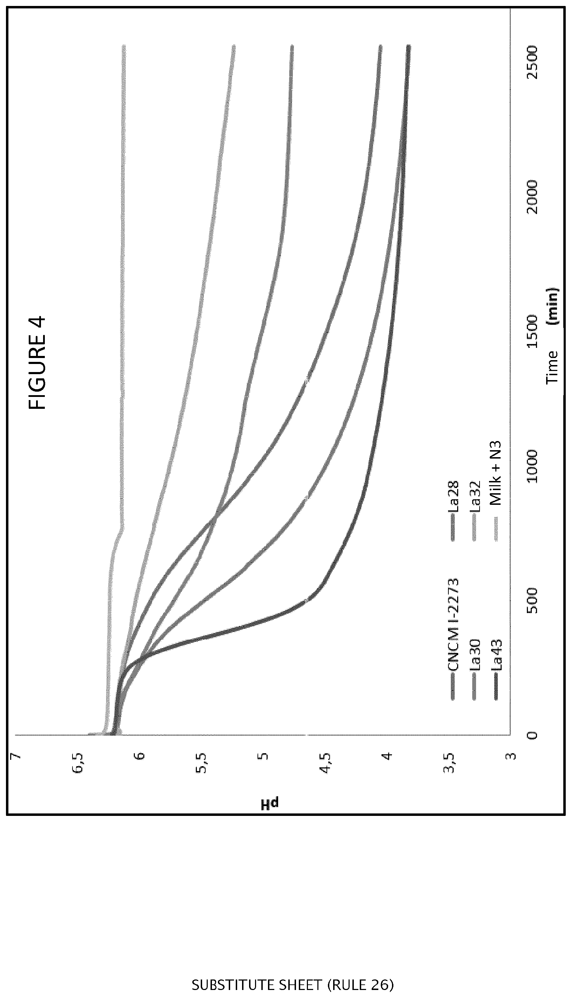

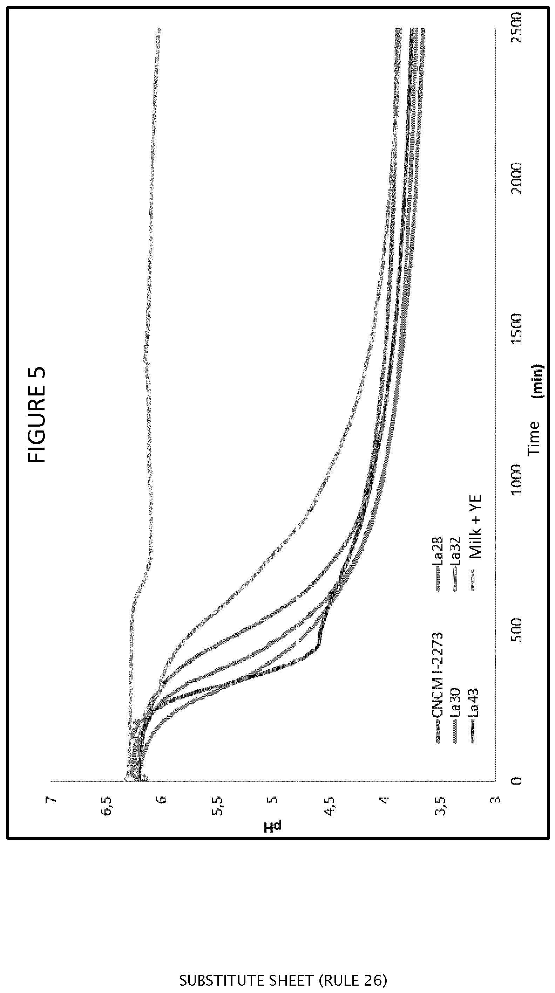

A screening of 5 strains of *L. acidophilus* was carried out to identify strains that were suitable for the preparation of fermented milk products based on their milk acidification properties. The test strains for screening purposes were provided by Danone: *L. acidophilus* strains reference nos. 28, 30, 32, 43 and CNCM I-2273. The test strains were tested in milk (Fig. 2), milk supplemented with 20g/l glucose (Fig. 3), milk supplemented with 0.5g/l N3 peptide (Vitalarmor 950) (Fig. 4), and milk supplemented with 2g/l yeast extract (Fig. 5).

Fermented milk test products were prepared by combining whole cow milk (3.2% protein content; autoclaved at 120°C 15 minutes for sterilization) with inoculum (pure strains of *L. acidophilus*) and fermenting at 37°C. The inoculum of *L. acidophilus* was in thawed frozen form provided from the Danone bacterial strain collection.

As can be seen from Figures 2-5, strain CNCM I-2273 had above-average milk acidification kinetics. This was unexpected in view of the present invention in WO 2010/058294 discussed above.

### Example 2: Fermented Milk Product Preparation

Fermented milk test products were prepared by combining cow milk having 3% protein and 3.4% fat with inoculum and fermenting.

Bacterial strains were provided in frozen form. The inoculum comprised a *S. thermophilus inoculum* together with one of the test *L. acidophilus* strains inoculum.

The *S. thermophilus* used F-DVS ST-BODY-4 is a commercially available strain from Chr. Hansen.

A commercially available *L. acidophilus* strain (Control strain) was used as a control. This strain is considered as having acceptable levels of D-lactate production as demonstrated by long-standing commercial use with no associated consumer illness or injury as well as FDA GRAS and EFSA QPS status. The inoculum of Control strain contained 6 x 10¹⁰ CFU/g.

Strain CNCM I-2273 was tested in liquid frozen (thawed) format: inoculum "ino 1" was prepared by propagation on a medium containing milk-derived components inoculum "ino 2" was prepared by propagation on a medium of milk-derived components. The inoculums had 3.1 x 10¹⁰ CFU/g (ino 1) and 2.6 x 10¹⁰ CFU/g (ino 2) in frozen format.

The strains were inoculated in the milk in the following volumes to ensure comparable amounts of the strains of *L. acidophilus:*
*S. thermophilus* 0.03% v/v
*L. acidophilus* Control strain 0.02% v/v
*L. acidophilus* CNCM I-2273 0.04% v/v

CNCM I-2273 was also tested with the addition of 0.008% v/v yeast extract in the milk mixture.

Fermentation was carried out at 42 °C and monitored using a CINAC pH probe. The resultant fermented milk was cooled and stored at 10°C for determination of strain viability and D-lactate content after 3 days of storage.

### Results

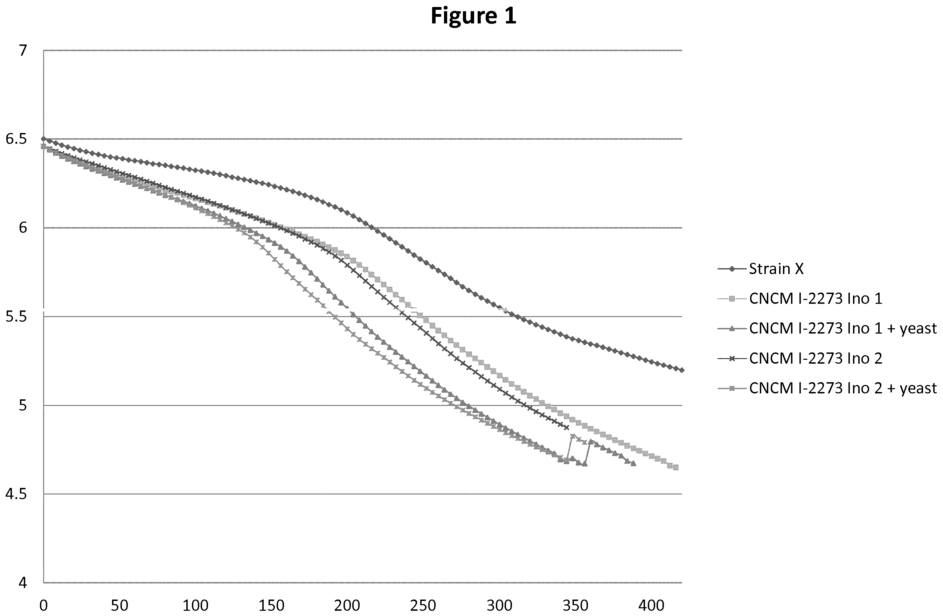

In all samples, D-lactate levels were detectable and lower than the target of 200 ppm. Figure 1 provides the acidification kinetics of the test products.

**Table 1: Characteristics of fermented milk products**

| | pH | fermentation time [H] | *L. acidophilus* Inoculation population [CFU/g] | *S. thermophilus* count Day 3 [CFU/g] | *L. acidophilus* count Day 3 [CFU/g] |
|---|---|---|---|---|---|
| Control strain | 5,04 | 08:20 | 1,20E+07 | 3,51E+07 | 2,68E+06 |
| CNCM I-2273 Frozen ino 1 | 4,69 | 07:10 | 1,24E+07 | 1,45E+08 | 1,84E+07 |
| CNCM I-2273 Frozen ino 1+ yeast extract | 4,69 | 05:58 | 1,24E+07 | 1,22E+08 | 1,78E+07 |
| CNCM I-2273 Frozen ino 2+ | 4,68 | 06:43 | 1,04E+07 | 1,28E+08 | 1,31 E+07 |
| CNCM I-2273 Frozen ino 2+ yeast extract | 4,68 | 06:08 | 1,04E+07 | 1,40E+09 | 1,65E+08 |

As can be seen from the above results: CNCM I-2273 produces acceptable levels of D-lactate, as does Control strain but at approximately one log higher population (CFU). CNCM I-2273 growth in milk (CFU count) was higher than the commercially used strain, especially when supplemented with yeast extract. This indicates that the D-lactate per CFU production is lower in CNCM I-2273 than Control strain.

CNCM I-2273 population was comparable between ino 1 and 2, however, some difference in D-lactate levels observed between different propagation conditions.

A trend towards symbiosis between CNCM I-2273 and *S. thermophilus* can be observed as ST-BODY-4's growth is impacted by a higher count of CNCM I-2273 (+1log for ST-BODY-4 in the last sample) and a faster fermentation time than was required for Control strain.

D-lactate levels in all the fermented milk products was comparable. However, taking into account that the population of CNCM I-2273 was significantly higher, it can be concluded that higher probiotic content can be achieved in fermented milk products with CNCM I-2273, while ensuring acceptable D-lactate levels or that the probiotic count can be reduced - with an expected reduction in D-lactate levels.

### Example 3: Confirmation of CFU/ D-lactate production

Fermented milks prepared according to Example 2. Multiple fermentations were carried out using the test (CNCM I-2273) and control strain both in combination with the ST-BODY-4 and in an alternative culture with an alternative "ST-Body" commercially available strain. Fermentation was carried out until bacterial counts in the ranges of 10⁶-10⁸ were achieved (at a pH of about 4.7) and D-lactate productions was measured.

A CFU/D-lactate relationship was confirmed, the higher the CFU the higher the D-lactate. It was also confirmed that D-lactate production of CNCM I-2273 was significantly lower per CFU compared to control strain.

Fermented milk could be produced with a CFU of up to 1x 10⁸ CFU/g, more preferably 2.5x 10⁷ CFU/g when using CNCM I-2273, however if using control strain the maximum CFU count achievable was 8 x 10⁶ CFU/g.

Thus a particularly interesting target range for having a high cfu/g probiotic product with good D-lactate levels would be within the range of at least 8x10⁶ cfu/g and up to 1x 10⁸ cfu/g. However, to ensure that margin of error is allowed the upper and limit may be set more conservatively (e.g. not more than 2.5, 5 or 7.5 x 10⁷ CFU/g *L. acidophilus* as the upper limit; at least 8.1, 8.3, 8.5 or 8.7 x 10⁶ or 1 x 10⁷ CFU/g as the lower limit).

## Claims

1. A fermented dairy composition comprising *L. acidophilus* CNCM I-2273, *S. thermophilus* and up to 0.02% w/w D-lactate and having a pH equal to or lower than 5.

2. A fermented dairy composition according to claim 1, suitable to be administered to infants or young children, preferably starting from the age of 4 months.

3. The fermented dairy composition according to any preceding claims comprising at least 8x 10⁶ CFU/g *L. acidophilus.*

4. A process for the preparation of a fermented dairy composition comprising fermenting a mixture comprising a dairy composition and *L. acidophilus* CNCM I-2273 and *S. thermophilus* to obtain a fermented dairy composition.

5. The process according to claim 4, wherein said fermented dairy composition comprises at least 8x 10⁶ CFU/g *L. acidophilus.*

6. The process according to claim 4 or 5, wherein said mixture further comprises at least one strain of *L. bulgaricus.*

7. The process according to any one of claims 4 to 6, wherein said fermentation is carried out at a temperature of less than about 42°C.

8. The process according to any one of claims 4 to 7, further comprising storing said fermented dairy composition for at least 73 hours prior to consumption.

## Patentansprüche

1. Fermentierte Milchproduktzusammensetzung, die *L. acidophilus* CNCM I-2273, S. *thermophilus* und bis zu 0,02 Gew.-% D-Laktat umfasst und einen pH-Wert von gleich oder niedriger als 5 aufweist.

2. Fermentierte Milchproduktzusammensetzung nach Anspruch 1, die zur Verabreichung an Kleinkindern oder kleinen Kinder, vorzugsweise ab einem Alter von 4 Monaten geeignet ist.

3. Fermentierte Milchproduktzusammensetzung nach einem der vorstehenden Ansprüche, die mindestens 8x 10⁶ CFU/g *L. acidophilusumfasst.*

4. Prozess zur Herstellung einer fermentierten Milchproduktzusammensetzung, umfassend Fermentieren einer Mischung, die eine Milchproduktzusammensetzung und *L. acidophilus* CNCM I- 2273 und *S. thermophilus* umfasst, um eine fermentierte Milchproduktzusammensetzung zu erhalten.

5. Prozess nach Anspruch 4, wobei die fermentierte Milchproduktzusammensetzung mindestens 8x 10⁶ CFU/g *L. acidophilus* enthält.

6. Prozess nach Anspruch 4 oder 5, wobei das Gemisch weiter Folgendes umfasst: mindestens einen Stamm von *L. bulgaricus.*

7. Prozess nach einem der Ansprüche 4 bis 6, wobei die Fermentation bei einer Temperatur von weniger als etwa 42 °C durchgeführt wird.

8. Prozess nach einem der Ansprüche 4 bis 7, weiter umfassend Lagern der fermentierten Milchproduktzusammensetzung für mindestens 73 Stunden vor Verbrauch.

## Revendications

1. Composition laitière fermentée comprenant *L. acidophilus* CNCM I-2273, S. thermophilus et jusqu'à 0,02 % p/p de D-lactate, et ayant un pH égal ou inférieur à 5.

2. Composition laitière fermentée selon la revendication 1, adaptée à être administrée à des nourrissons ou à de jeunes enfants, de préférence à partir de l'âge de 4 mois.

3. Composition laitière fermentée selon l'une quelconque des revendications précédentes, comprenant au moins 8 x 10⁶ UFC/g de L. acidophilus.

4. Procédé de préparation d'une composition laitière fermentée comprenant la fermentation d'un mélange comprenant une composition laitière et L. acidophilus CNCM I-2273 et S. thermophilus pour obtenir une composition laitière fermentée.

5. Procédé selon la revendication 4, dans lequel ladite composition laitière fermentée comprend au moins 8 x 10⁶ UFC/g de *L. acidophilus.*

6. Procédé selon la revendication 4 ou 5, dans lequel ledit mélange comprend en outre au moins une souche de *L. bulgaricus.*

7. Procédé selon l'une quelconque des revendications 4 à 6, dans lequel ladite fermentation est effectuée à une température inférieure à environ 42 °C.

8. Procédé selon l'une quelconque des revendications 4 à 7, comprenant en outre le stockage de ladite composition laitière fermentée pendant au moins 73 heures avant consommation.
